(19) Europäisches Patentamt
European Patent Office
Office européen des brevets

(11) **EP 3 801 194 B1**

(12) **EUROPÄISCHE PATENTSCHRIFT**

(45) Veröffentlichungstag und Bekanntmachung des Hinweises auf die Patenterteilung:
**01.10.2025 Patentblatt 2025/40**

(21) Anmeldenummer: **19728950.7**

(22) Anmeldetag: **04.06.2019**

(51) Internationale Patentklassifikation (IPC):
**A61B 3/028** (2006.01)　　　**A61B 3/103** (2006.01)

(52) Gemeinsame Patentklassifikation (CPC):
**A61B 3/028; A61B 3/103**

(86) Internationale Anmeldenummer:
**PCT/EP2019/064538**

(87) Internationale Veröffentlichungsnummer:
**WO 2019/234052 (12.12.2019 Gazette 2019/50)**

(54) **EINRICHTUNG ZUR PRÄZISIERUNG EINER SEHSCHÄRFENMESSUNG UND/ODER REFRAKTIONSBESTIMMUNG UND DEREN VERWENDUNG**

DEVICE FOR MAKING A VISUAL ACUITY MEASUREMENT AND/OR REFRACTION DETERMINATION MORE ACCURATE, AND USE THEREOF

DISPOSITIF POUR PRÉCISER UNE MESURE D'ACUITÉ VISUELLE ET/OU DÉTERMINATION DE RÉFRACTION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.06.2018 DE 102018113507**
**19.07.2018 DE 202018104158 U**

(43) Veröffentlichungstag der Anmeldung:
**14.04.2021 Patentblatt 2021/15**

(73) Patentinhaber: **Deutsche Augenoptik GmbH**
**75417 Mühlacker (DE)**

(72) Erfinder: **STELZER, Lars-Erik**
**75233 Tiefenbronn (DE)**

(74) Vertreter: **DREISS Patentanwälte PartG mbB**
**Friedrichstraße 6**
**70174 Stuttgart (DE)**

(56) Entgegenhaltungen:
WO-A1-2018/078409　　　US-A1- 2015 116 666
US-A1- 2017 119 247

**Beschreibung**

Die Erfindung betrifft eine Einrichtung zur Präzisierung von Sehschärfemessungen und/oder der Bestimmung der Refraktion sowie die zugehörige Verwendung einer solchen Einrichtung.

Bei einer Sehschärfenmessung wird die Sehschärfe (Visus) gemessen. Dies kann einerseits durch die Verwendung klassischer Sehzeichendarstellungen geschehen, die sich in einem gewissen Abstand zu einem Patienten befinden und dieser befragt wird, inwieweit er Elemente dieser Sehzeichendarstellung erkennen kann. Dabei kann eine Fehlsichtigkeit festgestellt werden. Andererseits können kompakte Einblickgeräte verwendet werden, um auf kleinem Raum gleichbleibende Beleuchtungsbedingungen herzustellen.

Weitere Verfahren verwenden Geräte, die das Auge vermessen und somit eine Fehlsichtigkeit feststellen können.

Unter Refraktion versteht man den Brechwert, den eine optische Korrektur besitzt, um im Auge ein scharfes Bild in unendlicher Entfernung zu erzeugen.

Bei einer Refraktionsbestimmung blickt der Patient durch ein Messgerät, beispielsweise einem Phoropter oder einer Messbrille, auf eine Sehzeichendarstellung. Um nun die genauen Korrekturlinsen zu bestimmen, werden in oder an dem Messgerät nach und nach Linsen eingefügt oder herausgenommen, bis die Fehlsichtigkeit ausgeglichen ist und der Patient möglichst kleine Elemente der Sehzeichendarstellung erkennen kann.

Hierbei befindet sich die Sehzeichendarstellung in einem endlichen Abstand zu dem Messgerät. Vorgeschrieben sind nach DIN 58220 in der Regel mindestens vier Meter, in der Praxis kann diese Entfernung auch durch Spiegelumlenkung zehn Meter oder mehr betragen.

Problematisch ist dabei, dass die Sehschärfe auf einem unendlichen Abstand definiert ist. Mit dem in der Regel zur Anwendung kommenden Abstand a von 4 bis 10 m können nur bedingt befriedigende Ergebnisse erzielt werden, da der Bereich von 4 bis 10 m dem unendlichen Abstand nur bedingt nahe kommt. Da sich die Sehzeichendarstellung nicht im Unendlichen befindet, akkommodiert das Auge auf die Refraktionsentfernung, was zur Folge hat, dass ein Refraktionsfehler entstehen kann.

Nach dem Stand der Technik wird der Refraktionsfehler entweder ignoriert oder es findet eine kursorische Nachbestimmung des Optikers und/oder des Augenarztes statt, indem der Patient, während er die zuvor bestimmten Linsen trägt, durch Blicken in die Ferne feststellt, inwieweit seine Sehschwäche korrigiert ist. Dies findet beispielsweise außerhalb des Optikgeschäfts oder der Arztpraxis statt oder durch eine sonstige freie Sicht in die Ferne, zum Beispiel durch ein Fenster.

Aus der US 2012/0154 742 A1 ist zur Refraktionsbestimmung die Verwendung eines elektro-optischen Phoropters bekannt.

Aus der US 2017/119247A1 und der US 2015/0116666A1 sind Einblickgeräte bekannt, bei denen die jeweilige Sehzeichendarstellung nur wenige Zentimeter vom Auge entfernt angeordnet ist. Aus der WO2018/078409A1 ist eine Brille bekannt, welche für jedes Auge ein Display aufweist.

Der vorliegenden Erfindung liegt die Aufgabe zugrunde, eine Einrichtung bereitzustellen, mit welcher der Refraktionsfehler minimiert oder gar ausgeschlossen wird.

Diese Aufgabe wird durch eine Einrichtung mit den Merkmalen des Anspruchs 1 gelöst.

Durch die Zusatzlinsenanordnung wird der Strahlengang bzw. werden die Strahlen im Strahlengang zwischen dem Auge, bzw. dessen Pupille und dem Messgerät, also einem Phoropter oder einer Messbrille, sphärisch ausgeglichen und parallelisiert, wodurch ein Effekt erzeugt wird, der bestünde, wenn der Abstand a ins Unendliche strebte. Ohne Verwendung wenigstens einer Zusatzlinsenanordnung kommt ein Refraktionsfehler in Höhe des Kehrwerts des Abstands zwischen Auge und Sehzeichendarstellung zu Stande. Folglich kann bei einem Abstand a von 4 Metern ein Refraktionsfehler von bis zu 0,25 dpt entstehen. Mittels der Verwendung wenigstens einer Zusatzlinsenanordnung kann der Refraktionsfehler sphärisch ausgeglichen werden. Somit ist es möglich, eine optische Korrektur, beispielsweise durch Brillengläser oder Kontaktlinsen, bereitzustellen, die optimal den Bedürfnissen des Patienten gerecht werden. Folglich ist es durch eine präzise Refraktionsbestimmung unter wenigstens weitgehender Beseitigung des Refraktionsfehlers möglich, trotz Sehschwäche auch Objekte, die sich insbesondere in weiter Entfernung des Patienten befinden, scharf zu sehen. Der Patient erhält also eine optische Korrektur, die seinen insbesondere physiologischen Bedürfnissen entspricht. Die Messung der Sehschärfe und/oder Refraktion erfolgt dabei für jedes Auge einzeln, oder auch gleichzeitig für beide Augen.

Diese Zusatzlinsenanordnung, die aus einer oder mehreren Linsen bestehen kann, ist hierbei so ausgestaltet, dass sie die Strahlen innerhalb des Strahlengangs zwischen dem Auge und dem Messgerät parallelisiert und damit einen Refraktionsfehler wenigstens weitgehend oder vollständig unterbindet. Insbesondere kann dies durch eine Zusatzlinsenanordnung, die mehrere Linsen aufweist oder auch durch eine einzelne geeignete Linse erfolgen.

Ferner ist es denkbar, eine gewisse Anzahl an Linsen bereitzustellen, die jeweils einen gewissen Bereich an Refraktionsfehlern zulassen, um handelsübliche Linsen zu verwenden.

Die wenigstens eine Zusatzlinsenanordnung und/oder Prismenanordnung ist insbesondere in und/oder an einem Phoropter oder einer Messbrille angeordnet. Hierbei ist es denkbar, bereits vorhandene Messgeräte nachzurüsten oder diese wenigstens eine Linse in und/oder an neue Geräte zu verbauen. Folglich ist es für den Verwender, beispielsweise einem Optiker oder Augenarzt, möglich, ohne Umgewöhnung oder größere Umstellung präzisere Ergebnisse zu erhalten.

Dabei ist vorgesehen, dass die Sehzeichendarstellung in einem Abstand a von mindestens 2 m, und vorzugsweise 4 m bis 10 m zu den Augen angeordnet ist. Dadurch ist eine Anpassung an die räumlichen Gegebenheiten und eine Untersuchung im Rahmen der DIN 58220 möglich.

Die Zusatzlinsenanordnung ist auf der der Sehzeichendarstellung zugewandten Seiten der Messanordnung angeordnet.

Erfindungsgemäß ist wenigstens eine Prismenanordnung vorgesehen, die das in die betrachtenden Augen fallende Licht der Sehzeichendarstellung derart umlenkt, dass die Fixierlinie des jeweiligen Auges wenigstens weitegehend durch die Mitte des Messgeräts und/oder parallel zur Fixierlinien des anderen Auges verläuft. Mit anderen Worten: die Fixierlinie des jeweiligen Auges verläuft dann senkrecht zur Linsenebene des Messeräts bzw. der Zusatzlinsenanordnung. Betrachten beide Augen gleichzeitig die Sehzeichendarstellung, dann verlaufen die Fixierlinien der beiden Augen wenigstens weitegehend parallel zueinander. Unter Fixierlinie wird dabei die Verbindungsgerade zwischen dem Austrittspunkt des Lichts aus der Prismenanordnung und der Mitte der Eintrittspupille des Auges und verstanden. Unter der Mitte des Messgeräts wird der Austrittspunkt des Lichts aus dem Messgerät verstanden, auf welchen die Person blicken muss, damit das Messgerät korrekte Messwerte liefert. Durch die Prismenanordnung erfolgt also ein prismatischer Ausgleich, der eine wenigstens weitgehende Parallelstellung der Augen gewährleistet. Ohne das Vorsehen der Prismenanordnung konvergieren die Fixierlinien der beiden Augen merklich hin zur Sehzeichendarstellung.

Bei einer Pupillendistanz PD der beiden Augen von beispielsweise PD = 66 mm weist jedes Auge eine Pupillendistanz zu einer gedachten Mittellinie von $PD_{R/L}$ = 33 mm auf. Bei einem Abstand a = 4 m zur Sehzeichendarstellung ergibt sich je Auge ein prismatischer Fehler von $F_{p\ R/L}$ = 0,825 cm/m, wobei gilt: $F_{p\ R/L} = PD_{R/L}\ /\ a$ = 0,825 cm/m.

Um den prismatischen Fehler $F_p$ ausgleichen zu können, ist folglich eine Prismenanordnung erforderlich, die bei jedem Auge den prismatischen Fehler von $F_{p\ R/L}$ wenigstens weitegehend ausgleicht. Idealerweise beträgt der prismatische Ausgleich p des jeweiligen Auges genau den Wert des prismatischen Fehlers $F_{p\ R/L}$.

Wird der sphärische Fehler ausgeglichen, und unterbleibt der prismatische Ausgleich, so hat dies eine unnatürliche Entkopplung von Vergenz und Akkommodation zur Folge. Zudem schaut die Person dann nicht lotrecht durch alle Linsen des Messgeräts, was zur Folge hat, dass Abbildungsfehler dieser Linsen zunehmen und eine Fehlsichtigkeit indizieren, die zwar bei der Refraktion in der Regel korrigiert wird, aber nicht genau der Fehlsichtigkeit der Person entspricht.

Durch den sphärischen Ausgleich der Zusatzlinsenanordnung und den prismatischen Ausgleich der Prismenanordnung werden die genannten Probleme behoben. Aufgrund des sphärischen Ausgleichs wird die Sehzeichendarstellung im Unendlichen abgebildet, unabhängig davon, wie groß der Abstand a tatsächlich ist. Somit sind auch kurze Abstände zur Sehzeichendarstellung ohne Abstriche an der Refraktionsqualität realisierbar. Aufgrund des prismatischen Ausgleichs wird eine prismatische Korrektion für Unendlich gemessen, ebenfalls unabhängig davon, wie groß der Abstand a tatsächlich ist. Werden der sphärische und der prismatische Ausgleich überlagert, führt dies zu einem synergetischen Effekt, der letztlich optimale Messwerte liefert.

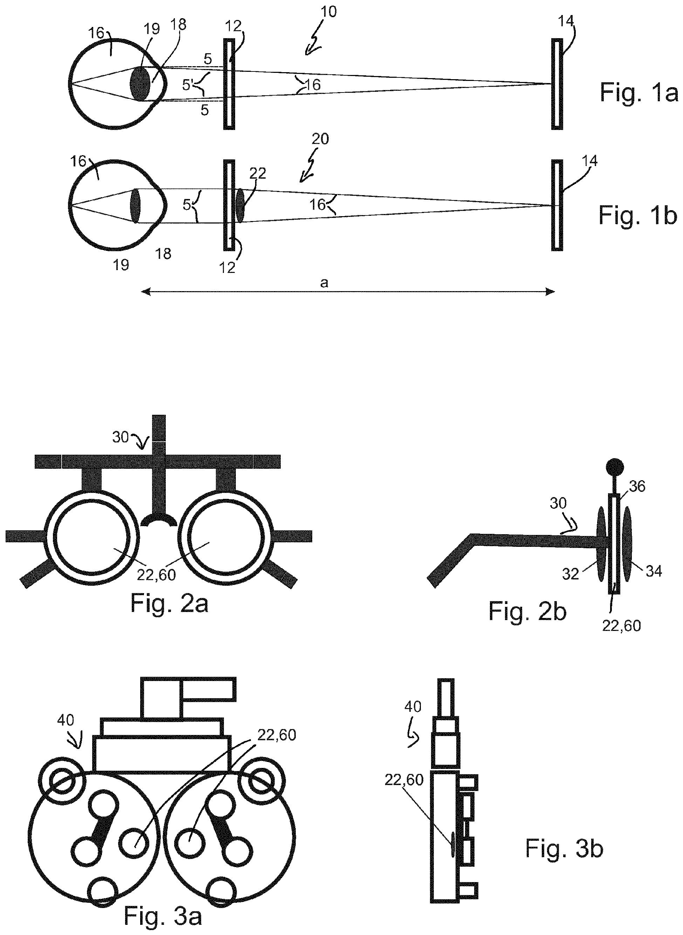

Die Prismenanordnung ist auf der der Sehzeichendarstellung zugewandten Seite der Zusatzlinsenanordnung angeordnet. Somit kann der Verwender, beispielsweise ein Optiker oder Augenarzt, die Zusatzlinsenanordnung und die Prismenanordnung an seine individuellen Bedürfnisse und Gegebenheiten des Mess- bzw. Bestimmungsortes anpassen. Insbesondere können die Anordnungen an das verwendete Messgerät angepasst werden. Eine beispielshafte erfindungsgemäße Anordnung innerhalb des Messgeräts lässt sich in Fig. 2 in einer Messbrille erkennen, eine beispielshafte erfindungsgemäße Anordnung unmittelbar hinter dem Messgerät, hier einem Phoropter, zeigt Fig. 3, wie weiter unten näher erläutert wird.

Erfindungsgemäß ist vorgesehen, dass die Zusatzlinsenanordnung auf der der Sehzeichendarstellung zugewandten Seite der Messanordnung und dass die Prismenanordnung auf der Sehzeichendarstellung zugewandten Seite der Zusatzlinsenanordnung vorgesehen ist. Aus Sicht des Patienten befindet sich dann das Messgerät unmittelbar vor seinen Augen, hinter dem Messgerät befindet sich die Zusatzlinsenanordnung und hinter der Zusatzlinsenanordnung befindet sich die Prismenanordnung. Durch eine derartige Anordnung kann insbesondere ein schädlicher Schrägblick verhindert werden.

Vorteilhafterweise kann ferner vorgesehen sein, dass die Zusatzlinsenanordnung und/oder die Prismenanordnung einstellbar ausgebildet sind. Zur Einstellung der Linsenanordnung kann die Brechkraft der Linsenanordnung verstellbar sein. Zur Einstellung der Prismenanordnung kann beispielsweise ein Prismenkompensator Verwendung finden. Diese Ausführungsform hat den Vorteil, dass die Zusatzlinsenanordnung und die Prismenanordnung zusammen mit dem Messgerät ausgeliefert werden kann und dass dann je nach der örtlichen Gegebenheiten eine Einstellung der Zusatzlinsenanordnung und der Prismenanordnung in Abhängigkeit des Abstands der Sehzeichendarstellung zum Messgerät und auch in Abhängigkeit der Pupillendistanz des jeweiligen Patienten erfolgen kann.

Als ebenfalls vorteilhaft hat sich herausgestellt, wenn die Zusatzlinsenanordnung und/oder die Prismenanordnung miteinander kombiniert in einer Optikeinheit untergebracht sind. Eine solche Optikeinheit kann dann vorzugsweise unmittelbar am Messgerät angeordnet und an die örtlichen Gegebenheiten angepasst werden.

Sinnvollerweise werden Zusatzlinsenanordnung verwendet, die eine Brechkraft b aufweist, die abhängig vom Abstand a zwischen Auge und Sehzeichendarstellung ist, und für die gilt: b = 1/a oder b = 1/a +/- **r,** wobei r eine etwaige Rundungstoleranz ist. Am vorteilhaftesten ist es, wenn die Brechkraft b = 1/a beträgt, da hierbei der Refraktionsfehler zu 100% ausgeglichen wird.

Um eine praktische Anwendung zu gewährleisten, kann es sinnvoll sein, Rundungstoleranzen r zuzulassen, so dass ein und dieselbe Linse für verschiedene Abstände a verwendet werden kann.

Denkbar ist es insbesondere, einen Bereich von Rundungstoleranzen von r = +/- 1/10 bis +/- 1/30 Dioptrie (dpt), insbesondere von r = +/- 1/20 dpt zuzulassen. Dies führt dazu, dass sich ein Refraktionsfehler bei einer Rundungstoleranz r = +/- 1/20 dpt auf höchstens 0,049 dpt beschränkt, so dass hierbei der Refraktionsfehler um 80% minimiert wird.

Die Rundungstoleranz r kann dabei so gewählt werden, dass für den Bereich von 4 bis 10 m lediglich 4 unterschiedliche Zusatzlinsenanordnungen, also Zusatzlinsenanordnung mit lediglich 4 unterschiedlichen Brechkräften b erforderlich sind, um den Refraktionsfehler $F_R$ ausreichend auszugleichen:

Für einen Abstand a von 4,00 m ergibt sich eine erste Zusatzlinsenanordnung, deren Brechkraft 0,25 dpt beträgt. Für einen Abstand von 4,10 m bis 5,10 m ergibt sich eine zweite Zusatzlinsenanordnung deren Brechkraft 0,2 dpt beträgt. Für einen Abstand von 5,20 m bis 6,90 m ergibt sich eine dritte Zusatzlinsenanordnung deren Brechkraft 0,15 dpt beträgt. Für einen Abstand von 7,00 m bis 10,00 m ergibt sich schließlich eine vierte Zusatzlinsenanordnung deren Brechkraft 0,1 dpt beträgt.

Sinnvollerweise werden Prismenanordnungen verwendet, die einen prismatischen Ausgleich p aufweisen, der nicht nur abhängig vom Abstand a ist, sondern auch vom Abstand PD der Pupillen, und für den dann gilt: $p = PD_{R/L} / a$ oder $p = PD_{R/L} /a$ +/- R, mit $PD_{R/L}$: halbe Pupillendistanz der Augen; R: Rundungstoleranz. Am vorteilhaftesten ist es, wenn der prismatischen Ausgleich $p = PD_{R/L} / a$ beträgt, da hierbei die ungewollte Konvergenz zu 100% ausgeglichen wird.

Um eine praktische Anwendung zu gewährleisten, kann es sinnvoll sein, Rundungstoleranzen R zuzulassen, so dass ein und dieselbe Prismenanordnung für verschiedene Abstände a verwendet werden kann.

Denkbar ist es insbesondere, für einen Abstand a von 4,00 m eine erste Prismenanordnung je Auge vorzusehen, deren prismatischer Ausgleich p = 0,65 cm/m beträgt. Für einen Abstand von 4,10 m bis 5,10 m ist sich eine zweite Prismenanordnung vorteilhaft, deren prismatischer Ausgleich p = 0,51 cm/m beträgt. Für einen Abstand von 5,20 m bis 6,90 m ergibt sich eine dritte Prismenanordnung je Auge, deren prismatischer Ausgleich p = 0,38 cm/m beträgt. Für einen Abstand von 7,00 m bis 10,00 m ergibt sich schließlich eine vierte Prismenanordnung je Auge, deren prismatischer Ausgleich p = 0,26 cm/m beträgt.

Des Weiteren kann die Zusatzlinsenanordnung und/oder die Prismenanordnung dauerhaft oder lösbar am Messgerät vorgesehen sein. Eine dauerhafte Anordnung hat den Vorteil, dass die einmalige Installation der Zusatzlinsenanordnung und der Prismenanordnung stets die gewünschte Korrektur ausübt, ohne dass die Gefahr besteht, durch beispielsweise Verrutschen oder Verschieben der Zusatzlinsenanordnung und/oder der Prismenanordnung das Ergebnis verfälscht wird. Ebenfalls kann hierbei die versehentliche Verwendung einer falschen Linse oder eines falschen Prismas ausgeschlossen werden.

Eine lösbare Anordnung hat den Vorteil, dass ein Messgerät in verschiedenen Situationen, beispielsweise verschiedenen Räumen oder verschiedenen Abständen zu einer Sehzeichendarstellung, verwendet werden kann und durch die Verwendung verschiedener Zusatzlinsenanordnung und/oder Prismenanordnungen dennoch ein optimales Ergebnis erzielt werden kann.

Sinnvollerweise befindet sich die wenigstens eine Zusatzlinsenanordnung und/oder Prismenanordnung zwischen dem Messgerät und der Sehzeichendarstellung oder in dem Messgerät selbst oder zwischen dem Patient und dem Messgerät. Ebenfalls ist eine Kombination dieser Möglichkeiten denkbar. Dies hat den Vorteil, dass auf die individuellen Bedürfnisse des Anwenders eingegangen werden kann.

Die hierbei beschriebene Einrichtung kann verwendet werden, um die Sehschärfe (Visus) des Patienten zu messen oder dessen Refraktion zu bestimmen. Die Verwendung hat den Vorteil, die bisher bestehende Ungenauigkeit auszugleichen.

Weitere Vorteile und vorteilhafte Ausgestaltungen der Erfindung ergeben sich aus nachfolgender Beschreibung, anhand derer die in den Figuren gezeigten Ausführungsformen näher beschrieben und erläutert werden.

Es zeigen:

Figur 1a    eine Einrichtung gemäß dem bekannten Stand der Technik,

Figur 1b    eine erfindungsgemäße Einrichtung,

Figur 2    eine Anordnung einer Zusatzlinsenanordnung an einer Messbrille,

Figur 3    eine Anordnung einer Zusatzlinsenanordnung an einem Phoropter,

Figur 4    eine schematische Draufsicht auf eine Einrichtung gemäß dem Stand der Technik,

Figur 5    eine schematische Draufsicht auf eine erfindungsgemäße Einrichtung, und

Figur 6    eine Zuordnung des Refraktionsfehlers und des prismatischen Fehlers und in Abhängigkeit des Abstands a und die Verwendung einer geeigneten Zusatzlinsenanordnung und Prismenanordnung.

In der Figur 1a ist eine Einrichtung 10 zur Sehschärfeprüfung und/oder zur Bestimmung einer Refraktion gemäß dem bekannten Stand der Technik gezeigt. Die Einrichtung 10 umfasst ein Messgerät 12 und eine Sehzeichendarstellung 14, wobei die Sehzeichendarstellung 14 in eine Abstand a zu einem menschlichen Auge 16, bzw. zu dessen Pupille 18 angeordnet ist und wobei sich das Messgerät 12 zwischen dem Auge 16 und der Sehzeichendarstellung 14 befindet.

Bei dem Messgerät 12 kann es sich beispielsweise um eine Messbrille, wie sie in Figur 2 gezeigt ist, oder um einen Phoropter, wie er in Figur 3 gezeigt ist, handeln.

Ferner ist der Strahlengang 16 des Lichts zwischen dem Messgerät 12 und der Sehzeichendarstellung 14 gezeigt. Der Strahlengang zwischen der Pupille 18 und dem Messgerät 12 ist in Figur 1a mit dem Bezugszeichen 5' gekennzeichnet; die Augenlinse mit 19. Der Strahlengang 5' ist bzw. die Strahlen im Strahlengang 5' sind dabei nicht parallel, sondern konvertieren geringfügig vom Auge 16 hin zum Messgerät 12. Da sich die Sehzeichendarstellung 14 nicht im Unendlichen befindet akkommodiert das Auge 16 auf die Refraktionsentfernung. Die Augenlinse 19 ist aufgrund der geringfügigen Nahanpassung in Fig. 1a etwas verdickt dargestellt; das Auge akkommodiert nicht "entspannt" ins Unendliche.

In der Figur 1b, die eine erfindungsgemäße Einrichtung 20 zeigt, ist am Messgerät 12 zusätzlich eine Zusatzlinsenanordnung 22 vorgesehen, beispielsweise in Form einer Linse. Durch die Zusatzlinsenanordnung 22 werden die Strahlen im Strahlengang 5 in Figur 1b zwischen der Pupille 18 und dem Messgerät 12 parallelisiert, wodurch ein Effekt erzeugt wird, der bestünde, wenn der Abstand a ins Unendliche rücken würde.

Die Brechkraft der Zusatzlinsenanordnung 22 zur Erzielung dieses Effekts berechnet sich aus dem Kehrwert des Abstandes **a.** Durch die Brechung der Zusatzlinsenanordnung 22 nimmt das betrachtende Auge 1 das Licht der Sehzeichendarstellung 14 folglich dahingehend war, dass der Abstand a ins Unendliche strebt. Dadurch nähert sich der Refraktionsfehler $F_R = 1/a$ dem Wert null. Das Auge 16 akkommodiert also gegen unendlich; die Augenlinse 19 ist in Figur 1b entsprechend dünn und entspannt. Bei einer Messung unter Verwendung der erfindungsgemäßen Einrichtung 20 kann folglich der Refraktionsfehler $F_R$ bzw. eine damit einhergehende unerwünschte Ungenauigkeit ausgeglichen werden.

Anders als in der Figur 1b gezeigt, ist es denkbar, dass sich die Zusatzlinsenanordnung 22 zwischen dem Messgerät 12 und dem Auge 16 befindet. Ebenfalls kann es sich um eine Kombination verschiedener Linsen handeln, die gemeinsam vor oder gemeinsam hinter oder aufgespalten vor und hinter dem Messgerät 12 befinden, wobei die Summe der Brechungsindizes im Idealfall 1/a oder 1/a +/- r beträgt. Eine weitere Ausführungsvariante kann vorsehen, dass sich eine oder mehrere Linsen innerhalb des Messgeräts 2 befinden.

In Figur 2a erkennt man die Frontansicht einer erfindungsgemäßen Messbrille 30, in Figur 2b die Seitenansicht. Hierbei erkennt man, dass sich die Zusatzlinsenanordnung 22 innerhalb des Gestells befindet. Es zeigt zudem Figur 2b, wie sich jeweils vor und hinter der Messbrille 30 Linsen 32, 34 befinden, die zur eigentlichen Refraktionsbestimmung variabel einsetzbar sind. Diese werden durch Halterungen 36 an der Messbrille 30 montiert. Die Zusatzlinsenanordnung 22 könnte aber auch an den Halterungen 36 montiert werden, um dem jeweiligen Modell oder den Bedürfnissen und Anforderungen des Anwenders, insbesondere Augenärzten oder Optikern, gerecht zu werden.

Es ist denkbar, die Zusatzlinsenanordnung 22 dauerhaft oder lösbar in oder an dem Gestell der Messbrille 30 vorzusehen. Die dauerhafte Anordnung hat den Vorteil, dass keine falschen Linsen verwendet werden können. Eine lösbare Anordnung hat den Vorteil, dass ein und dieselbe Brille 30 in Messkonstellationen mit variierendem Abstand a oder mit einem an den Augenabstand des Patienten angepassten Wert verwendet werden kann.

Figur 3a zeigt die Frontansicht eines erfindungsgemäßen Phoropters 40; Figur 3b stellt diesen in der Seitenansicht dar. In dieser Ausführungsvariante erkennt man, dass sich die Zusatzlinsenanordnung 22 vor dem Phoropter 40, also zwischen Phoropter 40 und Sehzeichendarstellung 14 befindet. Es ist jedoch ebenfalls denkbar, wenigstens eine Zusatzlinsenanordnung 22 innerhalb des Phoropters 40 und/oder hinter dem Gerät anzubringen. Hierbei kann dem jeweiligen Phoropter 40 den Bedürfnissen und Anforderungen des Anwenders gerecht werden. Es ist denkbar, die Zusatzlinsenanordnung 22 dauerhaft oder lösbar in oder an dem Phoropter 40 vorzusehen. Die dauerhafte Anordnung hat den Vorteil, dass keine falschen Linsen verwendet werden können. Eine lösbare Anordnung hat den Vorteil, dass ein und derselbe Phoropter 40 in Messkonstellationen mit variierendem Abstand a oder mit einem an den Augenabstand des Patienten angepassten Wert verwendet werden kann.

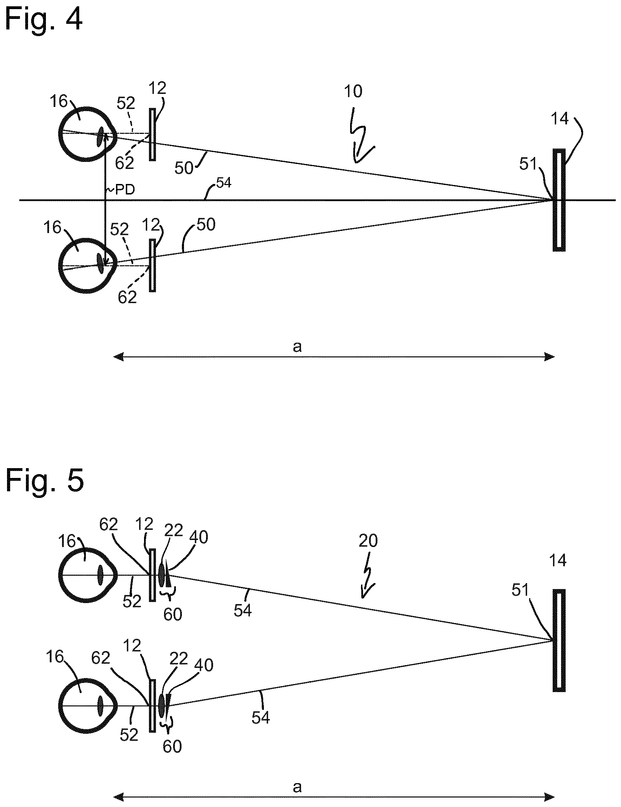

In der Figur 4 ist eine Einrichtung 10 gemäß dem bekannten Stand der Technik in schematischer Draufsicht gezeigt, bei der beide Augen 16 einer menschlichen Person dargestellt sind, die auf die Sehzeichendarstellung 14 blicken. Ferner sind die beiden Fixierlinien 50 gezeigt, die jeweils von der Mitte der Pupille 18 des jeweiligen Auges 16 hin zu einem auf der Sehzeichendarstellung 14 vorhandenen Objektpunkt 51 verläufen. Aufgrund des endlichen Abstands a konvergieren die beiden Fixierlinien 50 hin zum Objektpunkt 51.

Bei einer Pupillendistanz PD von beispielsweise PD = 66 mm weist jedes Auge eine Pupillendistanz $PD_{R/L}$ zu einer gedachten Mittellinie 54 von $PD_{R/L}$ = PD/2 = 33 mm auf. Bei einem Abstand a = 4 m zur Sehzeichendarstellung ergibt sich je Auge 16 ein prismatischer Fehler von $F_{p\ R/L}$ = 0,825 cm/m, wobei gilt:

$$F_{p\ R/L}\ =\ PD_{R/L}\ /\ a\ =\ 0,825\ cm/m\ ,$$

oder für beide Augen 16:

$$F_p\ =\ PD\ /\ a\ =\ 1,65\ cm/m\ .$$

Zudem schaut die Person dann nicht lotrecht durch das Messgerät 12 bzw. durch dessen Linsen, sondern an der Mitte 62 des Messgeräts 12 vorbei, was zur Folge hat, dass Abbildungsfehler dieser Linsen zunehmen und eine Fehlsichtigkeit indizieren.

Um dem abzuhelfen sieht die erfindungsgemäße Einrichtung 20, wie sie in Figur 5 in schematischer Draufsicht gezeigt ist, auf der dem Messgerät 12 abgewandten Seite der jeweiligen Zusatzlinsenanordnung 22 für jedes Auge 16 eine Prismenanordnung 40 vor. Die Prismenanordnungen 40 gleichen den prismatischen Fehler $F_p$ wenigstens weitgehend aus. Bei dem genannten Beispiel wird folglich bei jedem Auge 16 der prismatische Fehler von $F_{p\ R/L}$ = 0,825 cm/m idealerweise vollständig ausgleicht.

Die jeweilige Zusatzlinsenanordnung 22 und die zugehörige Prismenanordnung 40 können in einer gemeinsamen Optikeinheit 60 untergebracht sein. Die Optikeinheit 60 kann dabei, wie in den Figuren 2 und 3 gezeigt, an unterschiedlichen Orten an der Messbrille 30 oder dem Phoropter 40 untergebracht sein.

Die Prismenanordnungen 40 sind dabei so ausgebildet, dass das in das jeweils betrachtende Augen 16 fallende Licht der Sehzeichendarstellung 14 bzw. des Objektpunkts 51 der Sehzeichendarstellung 14 derart umgelenkt wird, dass die Fixierlinie 52 des jeweiligen Auges 16 durch die Mitte 62 des Messgeräts 12 verläuft. Die Fixierlinien 52 der beiden Augen 16 verlaufen also idealerweise senkrecht zur Linsenebene der Messeinrichtung 12 und der Zusatzeinrichtung 22 sowie parallel zueinander. Durch die Prismenanordnungen 40 erfolgt ein prismatischer Ausgleich p, der eine wenigstens weitgehende Parallelstellung der Augen 16 gewährleistet, wobei der Austrittspunkt des Lichts aus dem jeweiligen Messgerät 12 in der Mitte 62 des Messgeräts 12 liegt.

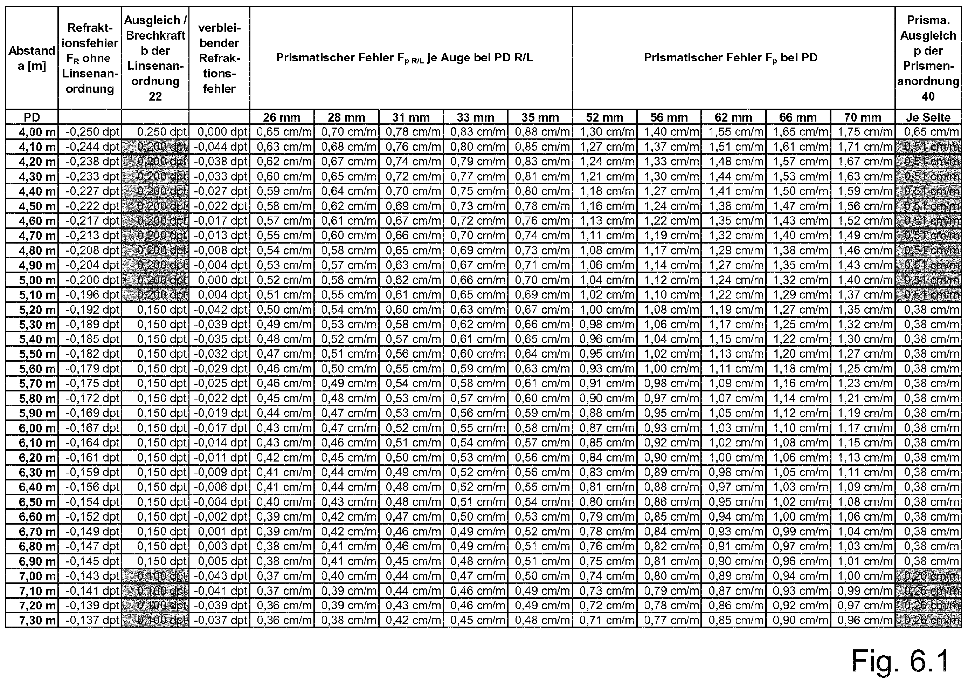

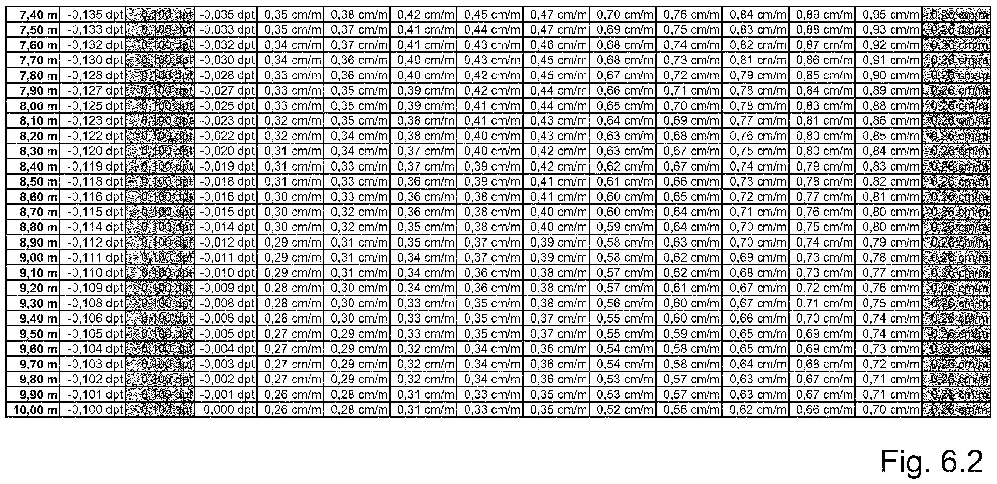

In der Tabelle gemäß Figur 6 (die in die Figuren 6.1 und 6.2 aufgeteilt ist) ist in der ersten Spalte der Abstand a vom Auge 16 bzw. der Pupille 18 zur Sehzeichendarstellung 14 in Metern aufgetragen. In der nächsten Spalte ist der Refraktionsfehler $F_R$ = 1/a für den jeweiligen Abstand a ohne das Vorsehen der Zusatzlinsenanordnung 22 in dpt sowie in der nächsten Spalte die Brechkraft von 4 unterschiedlichen Zusatzlinsenanordnung in dpt angegeben, wobei die unterschiedlichen Linsenanordnungen jeweils eine Brechkraft von 1/20 dpt Unterschied aufweisen. In der vierten Spalte ist der verbleibende, hinnehmbare Refraktionsfehler angegeben, also die Differenz zwischen dem Refraktionsfehler $F_R$ ohne Zusatzlinsenanordnung 22 und der Brechkraft b der jeweiligen Zusatzlinsenanordnung 22.

Daraus erkennt man, dass der Refraktionsfehler $F_R$ für einen Abstand a zwischen 4 m und 10 m Bereich von 0,1-0,25 dpt liegt und mit zunehmendem Abstand abnimmt. Es hat sich gezeigt, dass eine Zusatzlinsenanordnung ausreichend ist, deren Brechkraft sich durch Runden auf 1/20 dpt bestimmt. Dadurch sind für den Bereich von 4 bis 10 m lediglich 4 unterschiedliche Zusatzlinsenanordnungen, also Zusatzlinsenanordnung mit lediglich 4 unterschiedlichen Brechkräften b erforderlich, um den Refraktionsfehler $F_R$ ausreichend auszugleichen:
Für einen Abstand a von 4,00 m ergibt sich eine erste Zusatzlinsenanordnung, deren Brechkraft 0,25 dpt beträgt. Für einen Abstand von 4,10 m bis 5,10 m ergibt sich eine zweite Zusatzlinsenanordnung deren Brechkraft 0,2 dpt beträgt. Für einen Abstand von 5,20 m bis 6,90 m ergibt sich eine dritte Zusatzlinsenanordnung deren Brechkraft 0,15 dpt beträgt. Für einen Abstand von 7,00 m bis 10,00 m ergibt sich schließlich eine vierte Zusatzlinsenanordnung deren Brechkraft 0,1 dpt beträgt.

Folglich können mit nur vier unterschiedlichen Zusatzlinsenanordnungen alle Abstände a zwischen 4 und 10 Metern bedient werden, wobei sich der Refraktionsfehler $F_R$ bei einem Abstand a von 4,10 m auf höchstens 0,044 dpt reduziert. Ferner ist es denkbar, feinere Abstufungen der Linsen zu wählen, um ein noch präziseres Ergebnis zu erhalten.

In der Figur 6 sind in den weiteren Spalten die prismatischen Fehler je Auge $F_{p\ R/L}$ bei einer Pupillendistanz zu der Mittellinie 54 von 26 mm, 28 mm, 31 mm, 33 mm und 35 mm aufgetragen. Weiter rechts ist in der Tabelle der prismatische Fehler $F_p$ für einen Pupillenabstand PD der beiden Pupillen 18 für 52 mm, 46 mm, 62 mm, 66 mm und 70 mm aufgetragen. Bei Männern beträgt der Wert durchschnittlich 75 mm und bei Frauen von 72 mm.

Der prismatische Fehler für beide Augen 16 berechnet sich dabei wie folgt: $F_p$ = PD / **a**.

Um den prismatischen Fehler je nach Pupillendistanz PD und je nach Abstand a optimal ausgleichen zu können, kann insbesondere ein Prismenkompensator Verwendung finden.

Für den Fall, dass kein Prismenkompensator zur Verfügung steht, ist denkbar, im Bereich des Abstands a von 4-10 m vier unterschiedliche Prismenanordnungen 40 für das jeweilige Auge 16 vorzusehen, um innerhalb einer noch hinnehmbaren Toleranz zu bleiben. Es hat sich gezeigt, dass Prismenanordnungen ausreichend sind, deren prismatischer Ausgleich p 0,65 cm/m, 0,51 cm/, 0,38 cm/m oder 0,26 cm/m beträgt. Dadurch sind für den Bereich von 4 bis 10 m lediglich 4 unterschiedliche Prismenanordnungen erforderlich, um den prismatischen Fehler ausreichend auszugleichen:

Für einen Abstand a von 4,00 m ergibt sich eine erste Prismenanordnung je Auge 16, deren prismatischer Ausgleich p = 0,65 cm/m beträgt. Für einen Abstand von 4,10 m bis 5,10 m ergibt sich eine zweite Prismenanordnung, deren prismatischer Ausgleich p = 0,51 cm/m beträgt. Für einen Abstand von 5,20 m bis 6,90 m ergibt sich eine dritte Prismenanordnung je Auge 16, deren prismatischer Ausgleich p = 0,38 cm/m beträgt. Für einen Abstand von 7,00 m bis 10,00 m ergibt sich schließlich eine vierte Prismenanordnung je Auge 16, deren prismatischer Ausgleich p = 0,26 cm/m beträgt.

Optimalerweise können folglich 4 unterschiedliche Optikeinheiten 60 für einen Abstand a zwischen 4 m und 10 m bereitgestellt werden, die am oder im Messgerät angeordnet sein können:

Eine erste Optikeinheit findet dann bei einem Abstand a von 4 m Verwendung und weist eine Linsenanordnung mit einer Brechkraft von 0,25 dpt und eine Prismenordnung mit einem Ausgleich von 0,65 cm/m auf. Eine zweite Optikeinheit findet dann bei einem Abstand a von 4,10 m bis 5,10m Verwendung und weist eine Linsenanordnung mit einer Brechkraft von 0,2 dpt und eine Prismenanordnung mit einem Ausgleich von 0,51 cm/m auf. Eine dritte Optikeinheit findet bei einem Abstand a von 5,20 m bis 6,90 m Verwendung und weist eine Linsenanordnung mit einer Brechkraft von 0,15 dpt und eine Prismenanordnung mit einem Ausgleich von 0,38 cm/m auf. Eine vierte Optikeinheit findet bei einem Abstand a von 7,0 m bis 10 m Verwendung und weist eine Linsenanordnung mit einer Brechkraft von 0,1 dpt und eine Prismenanordnung mit einem Ausgleich von 0,26 cm/m auf.

Auch wenn im Ausführungsbeispiel lediglich auf den Abstand a zwischen 4 m und 10 m eingegangen wurde, können mit der Erfindung auch Abstände verwirklicht werden, die geringer als 4 m sind. Die Zusatzlinsenanordnung und die Prismenanordnung sind dann jeweils an den geringeren Abstand a angepasst auszubilden.

Durch Vorsehen solcher Optikeinheiten kann ein sphärischer und ein prismatischer Ausgleich erreicht werden, der letztlich zu optimalen Messwerten führt.

**Patentansprüche**

1. Einrichtung (20) zur Sehschärfeprüfung und/oder zur Bestimmung einer Refraktion wenigstens eines Auges (16) eines Patienten, mit einem Messgerät (12) in Form eines Phoropters (40) oder einer Messbrille (30), und mit einer Sehzeichendarstellung (14), wobei sich das Messgerät (12) zwischen den Augen (16) und der Sehzeichendarstellung (14) befindet, wobei wenigstens eine Zusatzlinsenanordnung (22) vorgesehen ist, die das in das jeweils betrachtende Auge (16) fallende Licht der Sehzeichendarstellung (14) mit einer Brechkraft derart bricht, dass für das betrachtende Auge (16) ein ins unendlich strebender Abstand zur Sehzeichendarstellung (14) wahrgenommen wird,

wobei die Einrichtung (20) wenigstens eine Prismenanordnung (40) umfasst, die dazu ausgebildet ist, das in das jeweils betrachtende Augen (16) des Patienten, dessen Sehschärfe geprüft und/oder gemessen wird, fallende Licht der Sehzeichendarstellung (14) derartumzulenken, dass die Fixierlinie (52) des jeweiligen Auges (16) des Patienten wenigstens weitgehend durch die Mitte des Messgeräts (12) und/oder parallel zur Fixierlinie (52) des anderen Auges (16) des Patienten verläuft,
**dadurch gekennzeichnet, dass** die Zusatzlinsenanordnung (22) auf der der Sehzeichendarstellung (14) zugewandten Seite der Messanordnung (12) und die Prismenanordnung (40) auf der der Sehzeichendarstellung (14) zugewandten Seite der Zusatzlinsenanordnung (22) angeordnet ist.

2. Einrichtung (20) nach Anspruch 1, **dadurch gekennzeichnet, dass** die Sehzeichendarstellung (14) in einem tatsächlichen Abstand a zu den Augen (16) des Patienten, dessen Sehschärfe geprüft und/oder gemessen wird, von mindestens 2 m, und vorzugsweise von 4 m bis 10 m zu den Augen angeordnet ist.

3. Einrichtung (20) nach einem der vorhergehenden Ansprüche, **dadurch gekennzeichnet, dass** für jedes Auge (16) eine Zusatzlinsenanordnung (22) und eine Prismenanordnung (40) vorgesehen ist.

4. Einrichtung (20) nach einem vorhergehenden Ansprüche, **dadurch gekennzeichnet, dass** die Prismenanordnung unmittelbar vor, innerhalb und/oder unmittelbar hinter dem Messgerät (12) vorgesehen ist.

5. Einrichtung (20) nach einem der vorhergehenden Ansprüche, **dadurch gekennzeichnet, dass** die Zusatzlinsenanordnung (22) und/oder die Prismenanordnung (40) einstellbar ausgebildet sind.

6. Einrichtung (20) nach einem der vorhergehenden Ansprüche, **dadurch gekennzeichnet, dass** die Zusatzlinsenanordnung (22) und/oder die Prismenanordnung (40) dauerhaft lösbar oder unlösbar am Messgerät (12) vorgesehen ist.

7. Einrichtung (20) nach einem der vorhergehenden Ansprüche,

**dadurch gekennzeichnet, dass** die Zusatzlinsenanordnung (22) eine Brechkraft b aufweist, für die gilt: b = 1/a oder b = 1/a +/- r, mit r: Rundungstoleranz; a:
tatsächlicher Abstand der Sehzeichendarstellung (14) zu den Augen (16) des Patienten, dessen Sehschärfe geprüft und/oder gemessen wird.

8. Einrichtung (20) nach Anspruch 7, **dadurch gekennzeichnet, dass** die Rundungstoleranz r im Bereich von r = +/- 1/10 bis +/- 1/30 Dioptrie liegt und weiter vorzugsweise 1/20 Dioptrie beträgt.

9. Einrichtung (20) nach einem der Ansprüche 3 bis 8, **dadurch gekennzeichnet, dass** die Prismenanordnung (40) für jedes Auge (16) des Patienten einen prismatischen Ausgleich p erreicht, für den gilt: p = $PD_{R/L}$ / a oder p = $PD_{R/L}$ /a +/- R, mit $PD_{R/L}$: halbe Pupillendistanz der Augen; R: Rundungstoleranz; a: tatsächlicher Abstand der Sehzeichendarstellung (14) zu den Augen (16) des Patienten, dessen Sehschärfe geprüft und/oder gemessen wird.

10. Einrichtung (20) nach Anspruch 9, **dadurch gekennzeichnet,**

   - **dass** die Optikeinheit je Auge (16) eine Zusatzlinsenanordnung (22) mit einer Brechkraft b = 0,25 dpt und eine Prismenanordnung (40) mit einem prismatischen Ausgleich p = 0,65 cm/m aufweist;
   - **dass** die Optikeinheit je Auge (16) eine Zusatzlinsenanordnung (22) mit einer Brechkraft b = 0,2 dpt und eine Prismenanordnung (40) mit einem prismatischen Ausgleich p = 0,51 cm/m aufweist;
   - **dass** die Optikeinheit je Auge (16) eine Zusatzlinsenanordnung (22) mit einer Brechkraft = 0,15 dpt und eine Prismenanordnung (40) mit einem prismatischen Ausgleich p = 0,38 cm/m aufweist; oder
   - **dass** die Optikeinheit je Auge (16) eine Zusatzlinsenanordnung (22) mit einer Brechkraft = 0,1 dpt und eine Prismenanordnung (40) mit einem prismatischen Ausgleich p = 0,26 cm/m aufweist.

11. Verwendung einer Einrichtung (20) nach einem der Ansprüche 1 bis 10 zur Sehschärfeprüfung und/oder zur Bestimmung einer Refraktion.

**Claims**

1. Apparatus (20) for testing visual acuity and/or for determining a refraction of at least one eye (16) of a patient, comprising a measuring device (12) in the form of a phoropter (40) or a trial frame (30), and comprising an optotype display (14), the measuring device (12) being located between the eyes (16) and the optotype display (14), at least one additional lens arrangement (22) being provided which refracts the light, incident in the relevant observing eye (16), from the optotype display (14) with a refractive power such that the observing eye (16) perceives a distance to the optotype display (14) that tends toward infinity,

   the apparatus (20) comprising at least one prism arrangement (40) which is designed to deflect the light of the optotype display (14), which light is incident in the relevant observing eye (16) of the patient whose visual acuity is being tested and/or measured, in such a way that the line of focus (52) of the relevant eye (16) of the patient runs at least largely through the center of the measuring device (12) and/or parallel to the line of focus (52) of the other eye (16) of the patient, **characterized in that** the additional lens arrangement (22) is arranged on the side of the measuring arrangement (12) facing the optotype display (14) and the prism arrangement (40) is arranged on the side of the additional lens arrangement (22) facing the optotype display (14).

2. Apparatus (20) according to claim 1, **characterized in that** the optotype display (14) is arranged at an actual distance a from the eyes (16) of the patient, whose visual acuity is being tested and/or measured, of at least 2 m, and preferably of 4 m to 10 m, from the eyes.

3. Apparatus (20) according to any of the preceding claims,
   **characterized in that** an additional lens arrangement (22) and a prism arrangement (40) are provided for each eye (16).

4. Apparatus (20) according to any of the preceding claims,
   **characterized in that** the prism arrangement is provided directly in front of, inside and/or directly behind the measuring device (12).

5. Apparatus (20) according to any of the preceding claims,
   **characterized in that** the additional lens arrangement (22) and/or the prism arrangement (40) are adjustable.

6. Apparatus (20) according to any of the preceding claims,
   **characterized in that** the additional lens arrangement (22) and/or the prism arrangement (40) is permanently detachably or non-detachably provided on the measuring device (12).

7. Apparatus (20) according to any of the preceding claims, **characterized in that** the additional lens arrangement (22) has a refractive power b for which the following applies: b = 1/a or b = 1/a +/- r, where r: rounding tolerance; a: actual distance from the optotype display (14) to the eyes (16) of the patient whose visual acuity is being tested and/or measured.

8. Apparatus (20) according to claim 7, **characterized in that** the rounding tolerance r is in the range of r = +/- 1/10 to +/- 1/30 diopter and more preferably 1/20 diopter.

9. Apparatus (20) according to any of claims 3 to 8, **characterized in that,** for each eye (16) of the patient, the prism arrangement (40) achieves a prismatic compensation p for which the following applies: p = $PD_{R/L}$ / a or p = $PD_{R/L}$ /a +/- R, where $PD_{R/L}$: half interpupillary distance of the eyes; R: rounding tolerance; a: actual distance from the optotype display (14) to the eyes (16) of the patient whose visual acuity is being tested and/or measured.

10. Apparatus (20) according to claim 9, **characterized in that,**

- for each eye (16), the optical unit comprises an additional lens arrangement (22) having a refractive power b = 0.25 dpt, and a prism arrangement (40) having a prismatic compensation p = 0.65 cm/m;
- for each eye (16), the optical unit comprises an additional lens arrangement (22) having a refractive power b = 0.2 dpt, and a prism arrangement (40) having a prismatic compensation p = 0.51 cm/m;
- for each eye (16), the optical unit comprises an additional lens arrangement (22) having a refractive power = 0.15 dpt, and a prism arrangement (40) having a prismatic compensation p = 0.38 cm/m; or
- for each eye (16), the optical unit comprises an additional lens arrangement (22) having a refractive power = 0.1 dpt, and a prism arrangement (40) having a prismatic compensation p = 0.26 cm/m.

11. Use of an apparatus (20) according to any of claims 1 to 10 for testing visual acuity and/or for determining a refraction.

**Revendications**

1. Dispositif (20) pour le contrôle de l'acuité visuelle et/ou pour la détermination d'une réfraction d'au moins un œil (16) qui regarde d'un patient, avec un appareil de mesure (12) sous forme d'un phoroptère (40) ou de lunettes de mesure (30), et avec une représentation d'optotype (14), dans lequel l'appareil de mesure (12) se trouve entre les yeux (16) et la représentation d'optotype (14), dans lequel au moins un ensemble de lentille supplémentaire (22) est prévu, qui réfracte la lumière de la représentation d'optotype (14) tombant dans l'œil (16) respectif qui regarde avec une puissance de réfraction telle que pour l'œil (16) qui regarde, une distance tendant vers l'infini est perçue par rapport à la représentation d'optotype (14), dans lequel le dispositif (20) comprend au moins un ensemble de prisme (40), qui est réalisé pour dévier la lumière de la représentation d'optotype (14) tombant dans l'œil (16) respectif du patient dont l'acuité visuelle est contrôlée et/ou mesurée, de telle sorte que la ligne de fixation (52) de l'œil (16) respectif du patient passe au moins en grande partie par le centre de l'appareil de mesure (12) et/ou parallèlement à la ligne de fixation (52) de l'autre œil (16) du patient, **caractérisé en ce que** l'ensemble de lentille supplémentaire (22) est disposé sur le côté de l'ensemble de mesure (12) tourné vers la représentation d'optotype (14) et l'ensemble de prisme (40) est disposé sur le côté de l'ensemble de lentille supplémentaire (22) tourné vers la représentation d'optotype (14).

2. Dispositif (20) selon la revendication 1, **caractérisé en ce que** la représentation d'optotype (14) est disposée à une distance effective a des yeux (16) du patient dont l'acuité visuelle est contrôlée et/ou mesurée, d'au moins 2 m, et de préférence de 4 m à 10 m des yeux.

3. Dispositif (20) selon l'une quelconque des revendications précédentes, **caractérisé en ce qu'**il est prévu pour chaque œil (16) un ensemble de lentille supplémentaire (22) et un ensemble de prisme (40).

4. Dispositif (20) selon l'une quelconque des revendications précédentes, **caractérisé en ce que** l'ensemble de prisme est prévu directement devant, à l'intérieur et/ou directement derrière l'appareil de mesure (12).

5. Dispositif (20) selon l'une quelconque des revendications précédentes, **caractérisé en ce que** l'ensemble de lentille supplémentaire (22) et/ou l'ensemble de prisme (40) sont réalisés de manière réglable.

6. Dispositif (20) selon l'une quelconque des revendications précédentes, **caractérisé en ce que** l'ensemble de lentille supplémentaire (22) et/ou l'ensemble de prisme (40) est prévu de manière à pouvoir être détaché ou à ne pas pouvoir être détaché de manière permanente de l'appareil de mesure (12).

7. Dispositif (20) selon l'une quelconque des revendications précédentes, **caractérisé en ce que** l'ensemble de lentille supplémentaire (22) présente une puissance de réfraction b pour laquelle s'applique : b = 1/a ou b = 1/a +/r, avec r : tolérance d'arrondi ; a : distance réelle entre la représentation d'optotype (14) et les yeux (16) du patient dont l'acuité visuelle est contrôlée et/ou mesurée.

8. Dispositif (20) selon la revendication 7, **caractérisé en ce que** la tolérance d'arrondi r se situe dans la plage de r = +/- 1/10 à +/- 1/30 dioptrie et est en outre de préférence de 1/20 dioptrie.

9. Dispositif (20) selon l'une quelconque des revendications 3 à 8, **caractérisé en ce que** l'ensemble de prisme (40) atteint pour chaque œil (16) du patient une compensation prismatique p pour laquelle s'applique : $p = PD_{R/L} / a$ ou $p = PD_{RL} /a+/- R$, avec $PD_{RL}$ : demi-distance pupillaire des yeux ; R : tolérance d'arrondi ; a : distance réelle entre la représentation d'optotype (14) et les yeux (16) du patient dont l'acuité visuelle est contrôlée et/ou mesurée.

10. Dispositif (20) selon la revendication 9, **caractérisé en ce**

 - **que** l'unité optique présente pour chaque œil (16) un ensemble de lentille supplémentaire (22) avec une puissance de réfraction b = 0,25 dpt et un ensemble de prisme (40) avec une compensation prismatique p = 0,65 cm/m ;
 - **que** l'unité optique présente pour chaque œil (16) un ensemble de lentille supplémentaire (22) avec une puissance de réfraction b = 0,2 dpt et un ensemble de prisme (40) avec une compensation prismatique p = 0,51 cm/m ;
 - **que** l'unité optique présente pour chaque œil (16) un ensemble de lentille supplémentaire (22) avec une puissance de réfraction de 0,15 dpt et un ensemble de prisme (40) avec une compensation prismatique p = 0,38 cm/m ; ou
 - **que** l'unité optique présente pour chaque œil (16) un ensemble de lentille supplémentaire (22) avec une puissance de réfraction = 0,1 dpt et un ensemble de prisme (40) avec une compensation prismatique p = 0,26 cm/m.

11. Utilisation d'un dispositif (20) selon l'une quelconque des revendications 1 à 10 pour le contrôle de l'acuité visuelle et/ou pour la détermination d'une réfraction.

Fig. 1a

Fig. 1b

Fig. 2a

Fig. 2b

Fig. 3a

Fig. 3b

Fig. 4

Fig. 5

| Abstand a [m] | Refrakt- ionsfehler $F_R$ ohne Linsenan- ordnung | Ausgleich / Brechkraft b der Linsenan- ordnung 22 | verblei- bender Refrak- tions- fehler | Prismatischer Fehler $F_{p\ R/L}$ je Auge bei PD R/L | | | | | Prismatischer Fehler $F_p$ bei PD | | | | | Prisma. Ausgleich p der Prismen- anordnung 40 |
|---|---|---|---|---|---|---|---|---|---|---|---|---|---|---|
| PD | | | | 26 mm | 28 mm | 31 mm | 33 mm | 35 mm | 52 mm | 56 mm | 62 mm | 66 mm | 70 mm | Je Seite |
| 4,00 m | -0,250 dpt | 0,250 dpt | 0,000 dpt | 0,65 cm/m | 0,70 cm/m | 0,78 cm/m | 0,83 cm/m | 0,88 cm/m | 1,30 cm/m | 1,40 cm/m | 1,55 cm/m | 1,65 cm/m | 1,75 cm/m | 0,65 cm/m |
| 4,10 m | -0,244 dpt | 0,200 dpt | -0,044 dpt | 0,63 cm/m | 0,68 cm/m | 0,76 cm/m | 0,80 cm/m | 0,85 cm/m | 1,27 cm/m | 1,37 cm/m | 1,51 cm/m | 1,61 cm/m | 1,71 cm/m | 0,51 cm/m |
| 4,20 m | -0,238 dpt | 0,200 dpt | -0,038 dpt | 0,62 cm/m | 0,67 cm/m | 0,74 cm/m | 0,79 cm/m | 0,83 cm/m | 1,24 cm/m | 1,33 cm/m | 1,48 cm/m | 1,57 cm/m | 1,67 cm/m | 0,51 cm/m |
| 4,30 m | -0,233 dpt | 0,200 dpt | -0,033 dpt | 0,60 cm/m | 0,65 cm/m | 0,72 cm/m | 0,77 cm/m | 0,81 cm/m | 1,21 cm/m | 1,30 cm/m | 1,44 cm/m | 1,53 cm/m | 1,63 cm/m | 0,51 cm/m |
| 4,40 m | -0,227 dpt | 0,200 dpt | -0,027 dpt | 0,59 cm/m | 0,64 cm/m | 0,70 cm/m | 0,75 cm/m | 0,80 cm/m | 1,18 cm/m | 1,27 cm/m | 1,41 cm/m | 1,50 cm/m | 1,59 cm/m | 0,51 cm/m |
| 4,50 m | -0,222 dpt | 0,200 dpt | -0,022 dpt | 0,58 cm/m | 0,62 cm/m | 0,69 cm/m | 0,73 cm/m | 0,78 cm/m | 1,16 cm/m | 1,24 cm/m | 1,38 cm/m | 1,47 cm/m | 1,56 cm/m | 0,51 cm/m |
| 4,60 m | -0,217 dpt | 0,200 dpt | -0,017 dpt | 0,57 cm/m | 0,61 cm/m | 0,67 cm/m | 0,72 cm/m | 0,76 cm/m | 1,13 cm/m | 1,22 cm/m | 1,35 cm/m | 1,43 cm/m | 1,52 cm/m | 0,51 cm/m |
| 4,70 m | -0,213 dpt | 0,200 dpt | -0,013 dpt | 0,55 cm/m | 0,60 cm/m | 0,66 cm/m | 0,70 cm/m | 0,74 cm/m | 1,11 cm/m | 1,19 cm/m | 1,32 cm/m | 1,40 cm/m | 1,49 cm/m | 0,51 cm/m |
| 4,80 m | -0,208 dpt | 0,200 dpt | -0,008 dpt | 0,54 cm/m | 0,58 cm/m | 0,65 cm/m | 0,69 cm/m | 0,73 cm/m | 1,08 cm/m | 1,17 cm/m | 1,29 cm/m | 1,38 cm/m | 1,46 cm/m | 0,51 cm/m |
| 4,90 m | -0,204 dpt | 0,200 dpt | -0,004 dpt | 0,53 cm/m | 0,57 cm/m | 0,63 cm/m | 0,67 cm/m | 0,71 cm/m | 1,06 cm/m | 1,14 cm/m | 1,27 cm/m | 1,35 cm/m | 1,43 cm/m | 0,51 cm/m |
| 5,00 m | -0,200 dpt | 0,200 dpt | 0,000 dpt | 0,52 cm/m | 0,56 cm/m | 0,62 cm/m | 0,66 cm/m | 0,70 cm/m | 1,04 cm/m | 1,12 cm/m | 1,24 cm/m | 1,32 cm/m | 1,40 cm/m | 0,51 cm/m |
| 5,10 m | -0,196 dpt | 0,200 dpt | 0,004 dpt | 0,51 cm/m | 0,55 cm/m | 0,61 cm/m | 0,65 cm/m | 0,69 cm/m | 1,02 cm/m | 1,10 cm/m | 1,22 cm/m | 1,29 cm/m | 1,37 cm/m | 0,51 cm/m |
| 5,20 m | -0,192 dpt | 0,150 dpt | -0,042 dpt | 0,50 cm/m | 0,54 cm/m | 0,60 cm/m | 0,63 cm/m | 0,67 cm/m | 1,00 cm/m | 1,08 cm/m | 1,19 cm/m | 1,27 cm/m | 1,35 cm/m | 0,38 cm/m |
| 5,30 m | -0,189 dpt | 0,150 dpt | -0,039 dpt | 0,49 cm/m | 0,53 cm/m | 0,58 cm/m | 0,62 cm/m | 0,66 cm/m | 0,98 cm/m | 1,06 cm/m | 1,17 cm/m | 1,25 cm/m | 1,32 cm/m | 0,38 cm/m |
| 5,40 m | -0,185 dpt | 0,150 dpt | -0,035 dpt | 0,48 cm/m | 0,52 cm/m | 0,57 cm/m | 0,61 cm/m | 0,65 cm/m | 0,96 cm/m | 1,04 cm/m | 1,15 cm/m | 1,22 cm/m | 1,30 cm/m | 0,38 cm/m |
| 5,50 m | -0,182 dpt | 0,150 dpt | -0,032 dpt | 0,47 cm/m | 0,51 cm/m | 0,56 cm/m | 0,60 cm/m | 0,64 cm/m | 0,95 cm/m | 1,02 cm/m | 1,13 cm/m | 1,20 cm/m | 1,27 cm/m | 0,38 cm/m |
| 5,60 m | -0,179 dpt | 0,150 dpt | -0,029 dpt | 0,46 cm/m | 0,50 cm/m | 0,55 cm/m | 0,59 cm/m | 0,63 cm/m | 0,93 cm/m | 1,00 cm/m | 1,11 cm/m | 1,18 cm/m | 1,25 cm/m | 0,38 cm/m |
| 5,70 m | -0,175 dpt | 0,150 dpt | -0,025 dpt | 0,46 cm/m | 0,49 cm/m | 0,54 cm/m | 0,58 cm/m | 0,61 cm/m | 0,91 cm/m | 0,98 cm/m | 1,09 cm/m | 1,16 cm/m | 1,23 cm/m | 0,38 cm/m |
| 5,80 m | -0,172 dpt | 0,150 dpt | -0,022 dpt | 0,45 cm/m | 0,48 cm/m | 0,53 cm/m | 0,57 cm/m | 0,60 cm/m | 0,90 cm/m | 0,97 cm/m | 1,07 cm/m | 1,14 cm/m | 1,21 cm/m | 0,38 cm/m |
| 5,90 m | -0,169 dpt | 0,150 dpt | -0,019 dpt | 0,44 cm/m | 0,47 cm/m | 0,53 cm/m | 0,56 cm/m | 0,59 cm/m | 0,88 cm/m | 0,95 cm/m | 1,05 cm/m | 1,12 cm/m | 1,19 cm/m | 0,38 cm/m |
| 6,00 m | -0,167 dpt | 0,150 dpt | -0,017 dpt | 0,43 cm/m | 0,47 cm/m | 0,52 cm/m | 0,55 cm/m | 0,58 cm/m | 0,87 cm/m | 0,93 cm/m | 1,03 cm/m | 1,10 cm/m | 1,17 cm/m | 0,38 cm/m |
| 6,10 m | -0,164 dpt | 0,150 dpt | -0,014 dpt | 0,43 cm/m | 0,46 cm/m | 0,51 cm/m | 0,54 cm/m | 0,57 cm/m | 0,85 cm/m | 0,92 cm/m | 1,02 cm/m | 1,08 cm/m | 1,15 cm/m | 0,38 cm/m |
| 6,20 m | -0,161 dpt | 0,150 dpt | -0,011 dpt | 0,42 cm/m | 0,45 cm/m | 0,50 cm/m | 0,53 cm/m | 0,56 cm/m | 0,84 cm/m | 0,90 cm/m | 1,00 cm/m | 1,06 cm/m | 1,13 cm/m | 0,38 cm/m |
| 6,30 m | -0,159 dpt | 0,150 dpt | -0,009 dpt | 0,41 cm/m | 0,44 cm/m | 0,49 cm/m | 0,52 cm/m | 0,56 cm/m | 0,83 cm/m | 0,89 cm/m | 0,98 cm/m | 1,05 cm/m | 1,11 cm/m | 0,38 cm/m |
| 6,40 m | -0,156 dpt | 0,150 dpt | -0,006 dpt | 0,41 cm/m | 0,44 cm/m | 0,48 cm/m | 0,52 cm/m | 0,55 cm/m | 0,81 cm/m | 0,88 cm/m | 0,97 cm/m | 1,03 cm/m | 1,09 cm/m | 0,38 cm/m |
| 6,50 m | -0,154 dpt | 0,150 dpt | -0,004 dpt | 0,40 cm/m | 0,43 cm/m | 0,48 cm/m | 0,51 cm/m | 0,54 cm/m | 0,80 cm/m | 0,86 cm/m | 0,95 cm/m | 1,02 cm/m | 1,08 cm/m | 0,38 cm/m |
| 6,60 m | -0,152 dpt | 0,150 dpt | -0,002 dpt | 0,39 cm/m | 0,42 cm/m | 0,47 cm/m | 0,50 cm/m | 0,53 cm/m | 0,79 cm/m | 0,85 cm/m | 0,94 cm/m | 1,00 cm/m | 1,06 cm/m | 0,38 cm/m |
| 6,70 m | -0,149 dpt | 0,150 dpt | 0,001 dpt | 0,39 cm/m | 0,42 cm/m | 0,46 cm/m | 0,49 cm/m | 0,52 cm/m | 0,78 cm/m | 0,84 cm/m | 0,93 cm/m | 0,99 cm/m | 1,04 cm/m | 0,38 cm/m |
| 6,80 m | -0,147 dpt | 0,150 dpt | 0,003 dpt | 0,38 cm/m | 0,41 cm/m | 0,46 cm/m | 0,49 cm/m | 0,51 cm/m | 0,76 cm/m | 0,82 cm/m | 0,91 cm/m | 0,97 cm/m | 1,03 cm/m | 0,38 cm/m |
| 6,90 m | -0,145 dpt | 0,150 dpt | 0,005 dpt | 0,38 cm/m | 0,41 cm/m | 0,45 cm/m | 0,48 cm/m | 0,51 cm/m | 0,75 cm/m | 0,81 cm/m | 0,90 cm/m | 0,96 cm/m | 1,01 cm/m | 0,38 cm/m |
| 7,00 m | -0,143 dpt | 0,100 dpt | -0,043 dpt | 0,37 cm/m | 0,40 cm/m | 0,44 cm/m | 0,47 cm/m | 0,50 cm/m | 0,74 cm/m | 0,80 cm/m | 0,89 cm/m | 0,94 cm/m | 1,00 cm/m | 0,26 cm/m |
| 7,10 m | -0,141 dpt | 0,100 dpt | -0,041 dpt | 0,37 cm/m | 0,39 cm/m | 0,44 cm/m | 0,46 cm/m | 0,49 cm/m | 0,73 cm/m | 0,79 cm/m | 0,87 cm/m | 0,93 cm/m | 0,99 cm/m | 0,26 cm/m |
| 7,20 m | -0,139 dpt | 0,100 dpt | -0,039 dpt | 0,36 cm/m | 0,39 cm/m | 0,43 cm/m | 0,46 cm/m | 0,49 cm/m | 0,72 cm/m | 0,78 cm/m | 0,86 cm/m | 0,92 cm/m | 0,97 cm/m | 0,26 cm/m |
| 7,30 m | -0,137 dpt | 0,100 dpt | -0,037 dpt | 0,36 cm/m | 0,38 cm/m | 0,42 cm/m | 0,45 cm/m | 0,48 cm/m | 0,71 cm/m | 0,77 cm/m | 0,85 cm/m | 0,90 cm/m | 0,96 cm/m | 0,26 cm/m |

Fig. 6.1

| | | | | | | | | | | | | | | |
|---|---|---|---|---|---|---|---|---|---|---|---|---|---|---|
| 7,40 m | -0,135 dpt | 0,100 dpt | -0,035 dpt | 0,35 cm/m | 0,38 cm/m | 0,42 cm/m | 0,45 cm/m | 0,47 cm/m | 0,70 cm/m | 0,76 cm/m | 0,84 cm/m | 0,89 cm/m | 0,95 cm/m | 0,26 cm/m |
| 7,50 m | -0,133 dpt | 0,100 dpt | -0,033 dpt | 0,35 cm/m | 0,37 cm/m | 0,41 cm/m | 0,44 cm/m | 0,47 cm/m | 0,69 cm/m | 0,75 cm/m | 0,83 cm/m | 0,88 cm/m | 0,93 cm/m | 0,26 cm/m |
| 7,60 m | -0,132 dpt | 0,100 dpt | -0,032 dpt | 0,34 cm/m | 0,37 cm/m | 0,41 cm/m | 0,43 cm/m | 0,46 cm/m | 0,68 cm/m | 0,74 cm/m | 0,82 cm/m | 0,87 cm/m | 0,92 cm/m | 0,26 cm/m |
| 7,70 m | -0,130 dpt | 0,100 dpt | -0,030 dpt | 0,34 cm/m | 0,36 cm/m | 0,40 cm/m | 0,43 cm/m | 0,45 cm/m | 0,68 cm/m | 0,73 cm/m | 0,81 cm/m | 0,86 cm/m | 0,91 cm/m | 0,26 cm/m |
| 7,80 m | -0,128 dpt | 0,100 dpt | -0,028 dpt | 0,33 cm/m | 0,36 cm/m | 0,40 cm/m | 0,42 cm/m | 0,45 cm/m | 0,67 cm/m | 0,72 cm/m | 0,79 cm/m | 0,85 cm/m | 0,90 cm/m | 0,26 cm/m |
| 7,90 m | -0,127 dpt | 0,100 dpt | -0,027 dpt | 0,33 cm/m | 0,35 cm/m | 0,39 cm/m | 0,42 cm/m | 0,44 cm/m | 0,66 cm/m | 0,71 cm/m | 0,78 cm/m | 0,84 cm/m | 0,89 cm/m | 0,26 cm/m |
| 8,00 m | -0,125 dpt | 0,100 dpt | -0,025 dpt | 0,33 cm/m | 0,35 cm/m | 0,39 cm/m | 0,41 cm/m | 0,44 cm/m | 0,65 cm/m | 0,70 cm/m | 0,78 cm/m | 0,83 cm/m | 0,88 cm/m | 0,26 cm/m |
| 8,10 m | -0,123 dpt | 0,100 dpt | -0,023 dpt | 0,32 cm/m | 0,35 cm/m | 0,38 cm/m | 0,41 cm/m | 0,43 cm/m | 0,64 cm/m | 0,69 cm/m | 0,77 cm/m | 0,81 cm/m | 0,86 cm/m | 0,26 cm/m |
| 8,20 m | -0,122 dpt | 0,100 dpt | -0,022 dpt | 0,32 cm/m | 0,34 cm/m | 0,38 cm/m | 0,40 cm/m | 0,43 cm/m | 0,63 cm/m | 0,68 cm/m | 0,76 cm/m | 0,80 cm/m | 0,85 cm/m | 0,26 cm/m |
| 8,30 m | -0,120 dpt | 0,100 dpt | -0,020 dpt | 0,31 cm/m | 0,34 cm/m | 0,37 cm/m | 0,40 cm/m | 0,42 cm/m | 0,63 cm/m | 0,67 cm/m | 0,75 cm/m | 0,80 cm/m | 0,84 cm/m | 0,26 cm/m |
| 8,40 m | -0,119 dpt | 0,100 dpt | -0,019 dpt | 0,31 cm/m | 0,33 cm/m | 0,37 cm/m | 0,39 cm/m | 0,42 cm/m | 0,62 cm/m | 0,67 cm/m | 0,74 cm/m | 0,79 cm/m | 0,83 cm/m | 0,26 cm/m |
| 8,50 m | -0,118 dpt | 0,100 dpt | -0,018 dpt | 0,31 cm/m | 0,33 cm/m | 0,36 cm/m | 0,39 cm/m | 0,41 cm/m | 0,61 cm/m | 0,66 cm/m | 0,73 cm/m | 0,78 cm/m | 0,82 cm/m | 0,26 cm/m |
| 8,60 m | -0,116 dpt | 0,100 dpt | -0,016 dpt | 0,30 cm/m | 0,33 cm/m | 0,36 cm/m | 0,38 cm/m | 0,41 cm/m | 0,60 cm/m | 0,65 cm/m | 0,72 cm/m | 0,77 cm/m | 0,81 cm/m | 0,26 cm/m |
| 8,70 m | -0,115 dpt | 0,100 dpt | -0,015 dpt | 0,30 cm/m | 0,32 cm/m | 0,36 cm/m | 0,38 cm/m | 0,40 cm/m | 0,60 cm/m | 0,64 cm/m | 0,71 cm/m | 0,76 cm/m | 0,80 cm/m | 0,26 cm/m |
| 8,80 m | -0,114 dpt | 0,100 dpt | -0,014 dpt | 0,30 cm/m | 0,32 cm/m | 0,35 cm/m | 0,38 cm/m | 0,40 cm/m | 0,59 cm/m | 0,64 cm/m | 0,70 cm/m | 0,75 cm/m | 0,80 cm/m | 0,26 cm/m |
| 8,90 m | -0,112 dpt | 0,100 dpt | -0,012 dpt | 0,29 cm/m | 0,31 cm/m | 0,35 cm/m | 0,37 cm/m | 0,39 cm/m | 0,58 cm/m | 0,63 cm/m | 0,70 cm/m | 0,74 cm/m | 0,79 cm/m | 0,26 cm/m |
| 9,00 m | -0,111 dpt | 0,100 dpt | -0,011 dpt | 0,29 cm/m | 0,31 cm/m | 0,34 cm/m | 0,37 cm/m | 0,39 cm/m | 0,58 cm/m | 0,62 cm/m | 0,69 cm/m | 0,73 cm/m | 0,78 cm/m | 0,26 cm/m |
| 9,10 m | -0,110 dpt | 0,100 dpt | -0,010 dpt | 0,29 cm/m | 0,31 cm/m | 0,34 cm/m | 0,36 cm/m | 0,38 cm/m | 0,57 cm/m | 0,62 cm/m | 0,68 cm/m | 0,73 cm/m | 0,77 cm/m | 0,26 cm/m |
| 9,20 m | -0,109 dpt | 0,100 dpt | -0,009 dpt | 0,28 cm/m | 0,30 cm/m | 0,34 cm/m | 0,36 cm/m | 0,38 cm/m | 0,57 cm/m | 0,61 cm/m | 0,67 cm/m | 0,72 cm/m | 0,76 cm/m | 0,26 cm/m |
| 9,30 m | -0,108 dpt | 0,100 dpt | -0,008 dpt | 0,28 cm/m | 0,30 cm/m | 0,33 cm/m | 0,35 cm/m | 0,38 cm/m | 0,56 cm/m | 0,60 cm/m | 0,67 cm/m | 0,71 cm/m | 0,75 cm/m | 0,26 cm/m |
| 9,40 m | -0,106 dpt | 0,100 dpt | -0,006 dpt | 0,28 cm/m | 0,30 cm/m | 0,33 cm/m | 0,35 cm/m | 0,37 cm/m | 0,55 cm/m | 0,60 cm/m | 0,66 cm/m | 0,70 cm/m | 0,74 cm/m | 0,26 cm/m |
| 9,50 m | -0,105 dpt | 0,100 dpt | -0,005 dpt | 0,27 cm/m | 0,29 cm/m | 0,33 cm/m | 0,35 cm/m | 0,37 cm/m | 0,55 cm/m | 0,59 cm/m | 0,65 cm/m | 0,69 cm/m | 0,74 cm/m | 0,26 cm/m |
| 9,60 m | -0,104 dpt | 0,100 dpt | -0,004 dpt | 0,27 cm/m | 0,29 cm/m | 0,32 cm/m | 0,34 cm/m | 0,36 cm/m | 0,54 cm/m | 0,58 cm/m | 0,65 cm/m | 0,69 cm/m | 0,73 cm/m | 0,26 cm/m |
| 9,70 m | -0,103 dpt | 0,100 dpt | -0,003 dpt | 0,27 cm/m | 0,29 cm/m | 0,32 cm/m | 0,34 cm/m | 0,36 cm/m | 0,54 cm/m | 0,58 cm/m | 0,64 cm/m | 0,68 cm/m | 0,72 cm/m | 0,26 cm/m |
| 9,80 m | -0,102 dpt | 0,100 dpt | -0,002 dpt | 0,27 cm/m | 0,29 cm/m | 0,32 cm/m | 0,34 cm/m | 0,36 cm/m | 0,53 cm/m | 0,57 cm/m | 0,63 cm/m | 0,67 cm/m | 0,71 cm/m | 0,26 cm/m |
| 9,90 m | -0,101 dpt | 0,100 dpt | -0,001 dpt | 0,26 cm/m | 0,28 cm/m | 0,31 cm/m | 0,33 cm/m | 0,35 cm/m | 0,53 cm/m | 0,57 cm/m | 0,63 cm/m | 0,67 cm/m | 0,71 cm/m | 0,26 cm/m |
| 10,00 m | -0,100 dpt | 0,100 dpt | 0,000 dpt | 0,26 cm/m | 0,28 cm/m | 0,31 cm/m | 0,33 cm/m | 0,35 cm/m | 0,52 cm/m | 0,56 cm/m | 0,62 cm/m | 0,66 cm/m | 0,70 cm/m | 0,26 cm/m |

Fig. 6.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120154742 A1
- US 2017119247 A1
- US 20150116666 A1
- WO 2018078409 A1